# EUROPEAN PATENT APPLICATION

(11) **EP 2 445 320 A1**
(43) Date of publication of application: **25.04.2012**
(21) Application number: 10013940.1
(22) Date of filing: 25.10.2010
(51) Int. Cl.: H05H 1/24, A61L 2/14, B01J 19/08

(54) **Energy harvesting cold atmospheric plasma generator**

(71) Applicant: Max-Planck-Gesellschaft zur Förderung der Wissenschaften e.V., 80539 München (DE)
(72) Inventor: Morfill, Gregor, 81927 Munich (DE)
(74) Representative: Gleiss & Grosse

(57) **Abstract**

The invention relates to a plasma applicator (1) for applying a non-thermal plasma, particularly for disinfection purposes. The plasma applicator (1) comprises an electrically driven plasma source (2) generating the non-thermal plasma and an electrical power source (3) providing electric energy, wherein the power source (3) in connected to the plasma source (2) and powers the plasma source (2). Further, the power source (3) comprises an energy harvesting device (4), wherein the energy harvesting device (4) converts ambient energy into the electrical energy needed for the operation of the plasma source (2). For example, a piezoelectric generator can be used for powering the plasma source (2).

## Description

### Field of the invention

The invention relates to a plasma applicator for application of a non-thermal plasma, particularly for disinfection purposes.

### Background of the invention

The use of a non-thermal, low-temperature plasma for the treatment of wounds and for disinfection purposes is disclosed, for example, in WO 2010/094304 A1, WO 2010/034451 A1, WO 2010/022871 A1, WO 2008/138504 A1, WO 2007/031250 A1 and WO 2010/094307 A1. These publications disclose plasma applicators comprising an electrically powered plasma source, wherein the electric energy needed for the operation of the plasma source is supplied either by mains or by an integrated battery.

However, mains-operated plasma applicators do not allow a mobile use of the plasma applicator and battery-powered plasma applicators have the disadvantage of a limited battery capacity and the need to accommodate a quite heavy and large battery.

### Summery of the invention

Therefore, it is a general object of the invention to provide an improved plasma applicator for applying a non-thermal, low-temperature plasma without a need for a battery or a connection to mains.

This object is achieved by a novel plasma applicator according to the main claim.

The invention is based on the general idea to use energy harvesting techniques to power the plasma source. This is advantageous since it eliminates the need for a battery or a connection to mains.

The plasma applicator according to the invention comprises an electrically driven plasma source generating the non-thermal plasma. The plasma source itself can be of a conventional type as disclosed, for example, in the aforementioned publications WO 2010/094304 A1, WO 2010/034451 A1, WO 2010/022871 A1, WO 2008/138504 A1, WO 2007/031250 A1 and WO 2010/094307 A1 which are therefore incorporated by reference herein. However, the invention is not restricted to the types of plasma sources disclosed in these publications. It is rather possible to employ other types of electrically driven plasma sources which are known in the state of the art. For example, the plasma source may comprise a dielectric barrier discharge (DBD) electrode arrangement or a surface micro discharge (SMD) electrode arrangement. Further, plasma bullets or plasma jets can be employed for generating the plasma.

Further, the plasma applicator according to the convention comprises an electric power source providing electric energy, wherein the power source is connected to the plasma source and powers the plasma source.

In contrast to the conventional plasma applicators, the power source is not a battery or a mains-connection. Instead, the power source comprises an energy harvesting device converting ambient energy into the electric energy needed for the operation of the electrically driven plasma source. The term energy harvesting is known as a process in which electric energy is derived from external sources, e.g. solar power, thermal energy, wind energy, salinity genetic energy. These types of energy harvesting devices are generally known in the state of the art.

For example, US 2006/0021261 A1 discloses an energy harvesting device in the form of a piezoelectric generator which is integrated in the heel of a piece of footwear so that the piezoelectric generator is actuated by footsteps thereby generating electric energy.

Another example of an energy harvesting device is a thermo-electric generator which uses temperature gradients and generates a corresponding thermo-voltage according to the well-known Seebeck effect.

However, the invention is not restricted to the afore-mentioned types of energy harvesting devices for powering the electrically driven plasma source. For example, it is conceivable to generate the electric energy needed for the operation of the plasma source by biomechanical harvesting, ambient-radiation sources, pyroelectric energy harvesting, electrostatic (capacitic) energy harvesting or blood sugar energy harvesting. However, the preferred embodiment of the invention uses a piezoelectric generator to power the plasma source.

In this connection, it should be noted that the energy harvesting device generally provides the electric energy in a format (e.g. voltage, pulse duration, frequency etc.) which is not directly suitable for powering the plasma source. Therefore, the power source preferably comprises a converter circuit which converts the electric power provided by the energy harvesting device in the power needed for operating the plasma source. For example, the converter circuit can be adapted to transform the voltage of the electrical energy. Further, the converter circuit can be adapted to accumulate the electrical energy provided by the energy harvesting device. Moreover, the converter circuit might comprise a rectifier. Suitable converter circuits are disclosed, for example, in US 2006/0021261 A1 which is therefore incorporated herein by reference.

In a preferred embodiment of the invention, the power source and/or the plasma source is at least partially arranged in a piece of clothing, particularly in a glove or in a coat, wherein the power source is preferably activated by movements of a person wearing the piece of clothing. For example, the afore-mentioned piezoelectric generator can be arranged in an armpit of the piece of clothing so that movements of the arms of the person wearing the piece of clothing actuate the piezoelectric generator thereby generating the electric energy needed for the operation of the plasma source. Further, the plasma source itself is preferably also arranged within the armpit of the piece of clothing so that the plasma generated by the plasma source disinfects the armpit of the piece of clothing thereby avoiding maloudor.

In another embodiment of the invention, the piezoelectric generator is at least partially arranged in a piece of footwear, particularly in a heel of a shoe. Such an arrangement is per se known in the state of the art, e.g. from US 2006/0021261 A1 so that this publication is incorporated by reference herein. The piezoelectric generator is preferably driven by footsteps of a person carrying the piece of footwear so that the biomechanical energy provided by the person wearing the piece of footwear is used for actuating the piezoelectric generator. In this embodiment, the plasma source is at least partially arranged in the piece of footwear, particularly in a sole of the piece of footwear that the plasma source disinfects the interior of the piece of footwear thereby avoiding maloudor and fungal infections.

In yet another embodiment of the invention, the piezoelectric generator and/or the plasma source is at least partially arranged in a manual operating element, particularly in a door knob, in a door handle or in a bathroom device, in particular with a movable component, like e. g. a toilet seat or part thereof. In this embodiment, a manual actuation of the manual operating element causes the piezoelectric generator to generate the electric energy needed for the operation of the plasma source. Further, the plasma source disinfects the surface of the manual operating element. Such an improved door handle could be used, for example, in hospitals to avoid infections caused by patients touching the door handles.

In yet another embodiment, the novel plasma applicator is substantially pin-shaped like a ball pen, wherein the plasma applicator comprises a piezoelectric generator. In this embodiment, the piezoelectric generator is preferably mechanically coupled by a mechanism to a manual operating element, wherein any movement of the operating element is mechanically transmitted to the piezoelectric generator, so that a manual actuation of the manual operating element causes the piezoelectric generator to generate the electric energy needed for the operation of the plasma source. The mechanism preferably allows an axial movement of the operating element along the pin-shaped plasma applicator like in a conventional ball pen. Moreover, the novel plasma applicator according to the invention can be used for treatment of insect bites (suppression of itching), inclusion in shoes (prevention of maloudor, protection against fungal infections), inclusion in sanitary clothing such as gloves and bandages to reduce transmission of diseases (in principle all textiles that require some amount of local disinfection), inclusion in door knobs and handles to disinfect these after each use (in principle all mechanically activated devices that require some amount of local disinfection) and for use in temporary dental pain relief by reducing bacterial load in cavities.

Further, it should be noted that the plasma applicator according to the invention is preferably mains-independent, mobile and/or hand-held.

Finally, it should be noted that the invention is not restricted to the afore-mentioned plasma applicator as a separate device but also encompasses the afore-mentioned articles (e.g. a piece of clothing, a piece of footwear, a manual operating element) comprising an integrated plasma applicator.

The invention and its particular features and advantages will become apparent from the following detailed description considered with reference to the accompanying drawings.

### Brief description of the drawings

Figure 1 shows a simplified drawing illustrating the general design of a plasma applicator according to the invention.
Figure 2 shows a cross-section of a shoe comprising an integrated plasma source for generating a low-temperature plasma and an integrated piezoelectric generator for powering the plasma source.
Figure 3A shows a cross-section of a pen-shaped plasma applicator comprising an integrated piezoelectric generator for powering the plasma source.
Figure 3B shows an enlarged and simplified cross section of the plasma source of the plasma applicator according to Figure 3A.
Figure 4 shows a coat comprising a integrated plasma applicators in the armpits of the coat.
Figure 5 shows a simplified schematic drawing illustrating a door handle comprising an integrated piezoelectric generator and an integrated plasma source.
Figure 6 shows a simplified schematic drawing illustrating a toilet seat comprising an integrated plasma applicator for disinfecting the toilet seat.

### Detailed description of the drawings

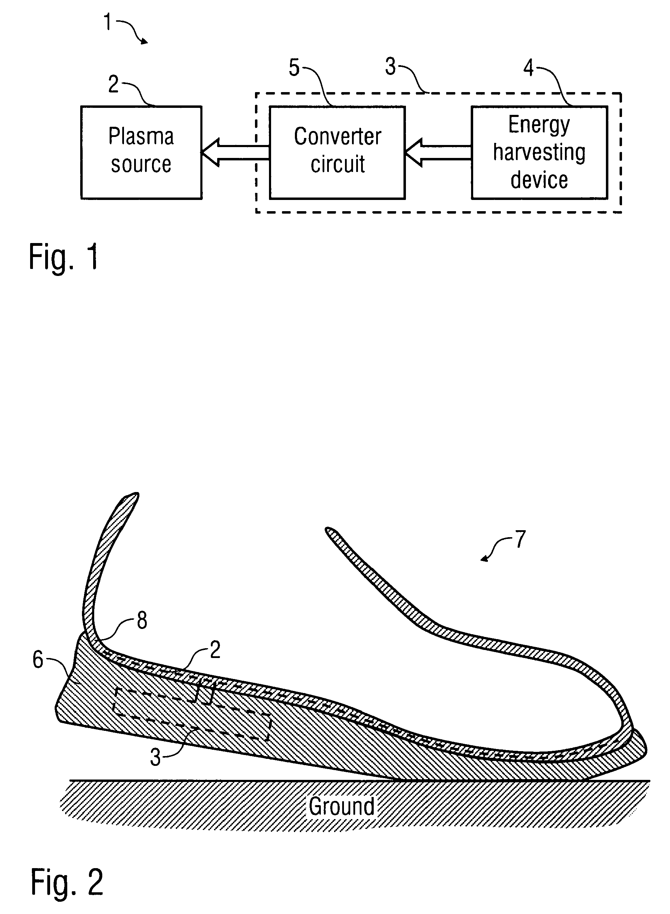

Figure 1 shows a schematic diagram illustrating the general design of a plasma applicator 1 according to the invention.

Firstly, the plasma applicator 1 comprises a conventional electrically driven plasma source 2 which generates a non-thermal, low-temperature plasma which is suitable for disinfection of wounds and other purposes. Suitable types of the plasma source 2 are disclosed, for example, in WO 2010/094304 A1, WO 2010/034451 A1, WO 2010/022871 A1, WO 2008/138504 A1, WO 2007/031250 A1 and WO 2010/094307 A1. However, the plasma source 2 can be of any other suitable type.

Further, the plasma applicator 1 according to the invention comprises a power source 3 providing the electric energy needed for the operation of the plasma source 2.

The power source 3 comprises an energy harvesting device 4 instead of a battery or a mains connection as in conventional plasma applicators. The energy harvesting device 4 can be, for example, a piezoelectric generator or a thermo-electric generator.

Further, the power source 3 comprises a converter circuit 5 which converts the electric energy provided by the energy harvesting device 4 into the electric energy needed for the operation of the plasma source. The converter circuit 5 is generally necessary since the energy harvesting device 4 typically does not provide the electric energy in a form suitable for powering the plasma source 3. For example, a rectifier and a buffer can be included in the converter circuit 5.

The novel plasma applicator 1 can be used for disinfecting surfaces as disclosed in, for example, WO 2010/094304 A1, WO 2010/034451 A1, WO 2010/022871 A1, WO 2008/138504 A1, WO 2007/031250 A1 and WO 2010/094307 A1.

Figure 2 shows a cross section of a preferred embodiment of the invention, wherein the energy harvesting device 4 is realized by a piezoelectric generator which is integrated in a heel 6 of shoe 7. Therefore, each footstep of the shoe 7 causes the piezoelectric generator in the energy harvesting device 4 to generate electric energy which is temporally stored by the converter circuit 5, wherein the converter circuit 5 is also integrated in the heel 6 of the shoe 7.

Further, the plasma source 2 is realized by a dielectric barrier discharge (DBD) electrode arrangement being integrated in the sole 8 of the shoe 7, so that the plasma source 2 generates the low-temperature plasma within the shoe 7 thereby avoiding any maloudor. The design of the dielectric barrier discharge (DBD) electrode arrangement is disclosed, for example, in WO 2010/094304 A1 which is therefore incorporated by reference herein. However, other types of plasma sources 2 can used, as well.

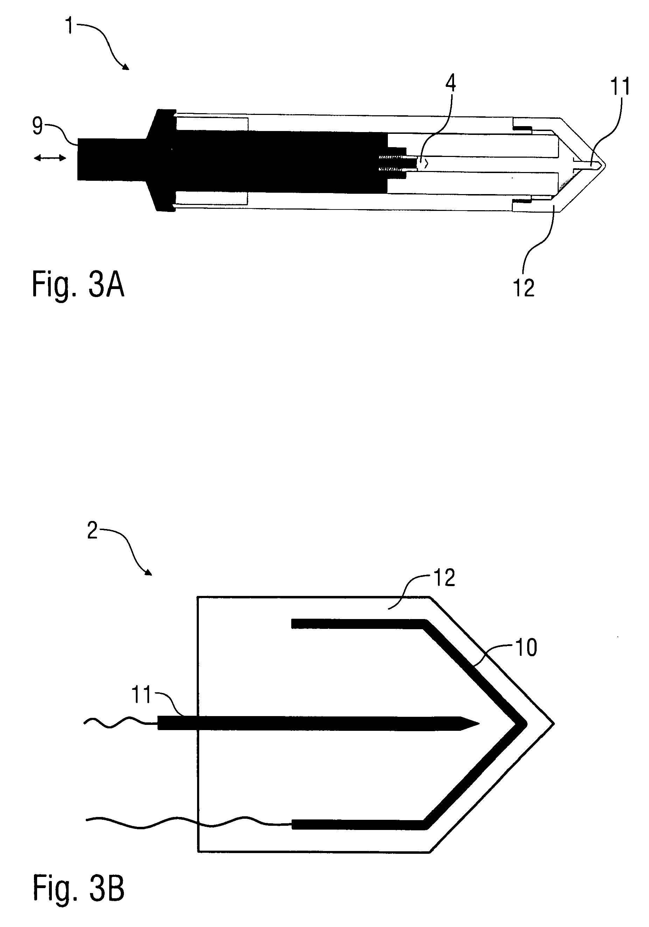

Figures 3A and 3B illustrate another embodiment of a plasma applicator 1 according to the invention, wherein the plasma applicator 1 is substantially pin-shaped and comprises in axially movable operating element 9 which acts on the energy harvesting device 4 in the form of a piezoelectric generator. Therefore, each movement of the operating element 9 by an operator causes the piezoelectric generator to generate electric energy.

At the tip of the pen-shaped plasma applicator 1, a plasma source 2 is integrated, wherein the plasma source 2 substantially consists of two electrodes 10, 11 which are embedded in a dielectric 12 as shown in Figure 3B.

The type of plasma applicator 1 as shown in Figures 3A and 3B can be used, for example, in temporary dental pain relief by reducing bacterial load in cavities.

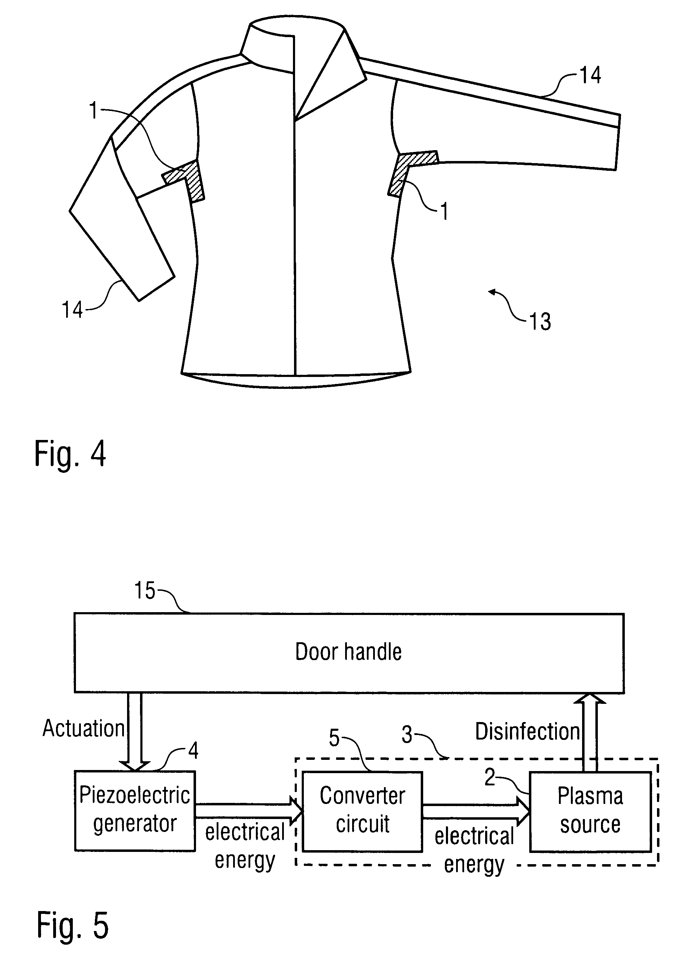

Figure 4 shows an other embodiment of a plasma applicator 1 which is arranged in the armpits of a coat 13, wherein the plasma applicators 1 each comprise a piezoelectric generator which is actuated by any movements of arms 14 of the coat 13. In other words, any arm movements of the person wearing the coat 13 activate the piezoelectric generators of the plasma applicators 1, so that the piezoelectric generators provide the electric energy needed for the operation of the plasma sources which are also arranged in the armpits of the coat 13 thereby avoiding any maloudor caused by sweat.

Finally, Figure 5 illustrates another embodiment of a plasma applicator 1 according to the invention, wherein the energy harvesting device 4 in the form of a piezoelectric generator is mechanically actuated by a door handle 15, so that each movement of the door handle 15 causes the piezoelectric generator to provide electric energy to the converter circuit 5.

Further, the plasma source 2 is arranged in such way that the low-temperature plasma is provided to the surface of the door handle 15 thereby disinfecting the surface of the door handle 15.

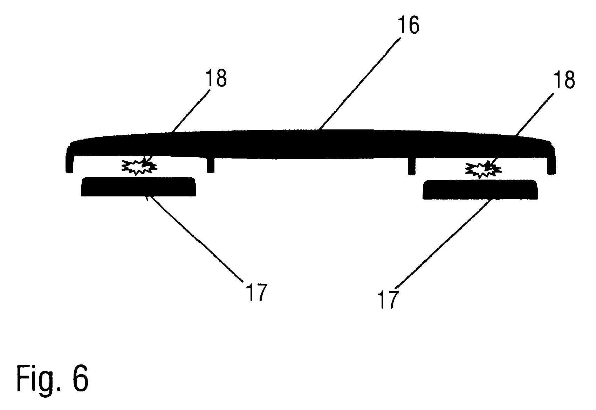

Finally, Figure 6 shows a schematic view of another embodiment of the invention, wherein a plasma applicator (not shown) according to the invention is embedded into a cover 16 of a toilet seat 17, so that a non-thermal plasma 18 is triggered between the cover 16 and the toilet seat 17 thereby disinfecting the surface of the toilet seat 16.

The plasma applicator comprises a piezoelectric generator which is mechanically coupled to the cover 16, so that a closing movement of the cover 16 actuates the piezoelectric generator thereby generating the plasma.

When the cover 16 is closed, the charge is released in the form of cold atmospheric plasma using an encapsulated electrode arrangement also located in the cover 16. The cover 16 is shaped to trap the plasma thus ensuring maximum bactericidal efficiency by using the afterglow as well.

Alternatively, the piezoelectric generator could also be coupled to the movable toiled seat 17.

Although the invention has been described with reference to the particular arrangement of parts, features and the like, these are not intended to exhaust all possible arrangements of features, and indeed many other modifications and variations will be ascertainable to those of skill in the art.

### List of reference numerals

- 1: Plasma applicator
- 2: Plasma source
- 3: Power source
- 4: Energy harvesting device
- 5: Converter circuit
- 6: Heel
- 7: Shoe
- 8: Sole
- 9: Operating element
- 10: Electrode
- 11: Electrode
- 12: Dielectric
- 13: Coat
- 14: Arms
- 15: Door handle
- 16: Cover
- 17: Toilet seat
- 18: Plasma

## Claims

1. Plasma applicator (1) for applying a non-thermal plasma, comprising:
a) an electrically driven plasma source (2) generating the non-thermal plasma, and
b) an electrical power source (3) providing electric energy, wherein the power source (3) in connected to the plasma source (2) and powers the plasma source (2), **characterized in that**
c) the power source (3) comprises an energy harvesting device (4), wherein the energy harvesting device (4) converts ambient energy into the electrical energy needed for the operation of the plasma source (2).

2. Plasma source according to claim 1, wherein the energy harvesting device (4) is selected from a group consisting of:
a) a piezoelectric generator (4),
b) a thermo-electric generator.

3. Plasma applicator (1) according to any of the preceding claims, wherein the power source (3) comprises a converter circuit (5) which converts the electrical power provided by the energy harvesting device (4) in the power needed for operating the plasma source (2).

4. Plasma applicator (1) according to any of the preceding claims, wherein
a) the power source (3) and/or the plasma source (2) is at least partially arranged in a piece of clothing (13), particularly in a glove or a coat (13), and/or
b) the power source (3) is activated by movements of a person wearing the piece of clothing (13), and/or
c) the plasma source (2) disinfects at least a portion of the piece of clothing (13).

5. Plasma applicator (1) according to any of claims 1 to 3, wherein
a) the piezoelectric generator (4) is at least partially arranged in a piece of footwear (7), particularly in a heel (6) of a shoe (7), and/or
b) the piezoelectric generator (4) is driven by footsteps of a person carrying the piece of footwear (7), and/or
c) the plasma source (2) is at least partially arranged in a piece of footwear (7), particularly in a sole (8) of the piece of footwear (7), and/or
d) the plasma source (2) disinfects the interior of the piece of footwear (7).

6. Plasma applicator (1) according to any of claims 1 to 3, wherein
a) the piezoelectric generator (4) and/or the plasma source (2) is at least partially arranged in a manual operating element (15), particularly in a door knob or in a door handle (15) and/or
b) a manual actuation of the manual operating element (15) causes the piezoelectric generator (4) to generate the electrical energy needed for the operation of the plasma source (2), and/or
c) the plasma source (2) disinfects the surface of the manual operating element (15).

7. Plasma applicator (1) according to any of the preceding claims, wherein
a) the plasma applicator (1) is substantially pin-shaped,
and/or
b) the plasma applicator (1) comprises a piezoelectric generator (4), and/or
c) the piezoelectric generator (4) is mechanically coupled by a mechanism to an operating element (9), which is movable and manually operable, wherein the mechanism transmits a manual movement of an operator to the piezoelectric generator (4) so that the piezoelectric generator (4) generates the electric energy needed for the operation of the plasma source (2), and/or.
d) the mechanism allows an axial movement of the operating element (9) along the pin-shaped plasma applicator (1).

8. Plasma applicator (1) according to any of the preceding claims, wherein the plasma applicator (1) is
a) mains-independent.
b) mobile and/or
c) hand-held.

9. Article, particularly a piece of clothing (13), a piece of footwear (7) or a manual operating element (15), comprising an integrated plasma applicator (1) according to any of the preceding claims.

10. Article according to claim 9, wherein
a) the article is a piece of clothing (13) comprising arms (14), wherein the plasma source (2) is arranged in an armpit of the piece of clothing (13), and/or
b) the piezoelectric generator (4) is actuated by movements of the arms (13) of the piece of clothing (13), or
c) the article is a toilet seat (17) or a cover (16) of a toilet seat (17), wherein the plasma applicator is embedded into the cover (16) or into the toilet seat (17), so that the non-thermal plasma disinfects the toilet seat (17) and/or the cover (16).

11. Article according to claim 10, wherein the piezoelectric generator of the plasma applicator is coupled to the cover (16) and/or to the toilet seat (17), so that a movement of the cover (16) or the toilet seat (17) actuates the piezoelectric generator.

12. Use of a plasma applicator (1) or an article according to any of the preceding claims:
(a) for a treatment of insect bites, and/or
(b) for temporary dental pain relief.
